(19) Europäisches Patentamt
European Patent Office
Office européen des brevets

(11) Veröffentlichungsnummer : **0 374 653 B1**

# (12) EUROPÄISCHE PATENTSCHRIFT

(45) Veröffentlichungstag der Patentschrift :
**09.09.92 Patentblatt 92/37**

(51) Int. Cl.$^5$ : **C07C 273/18,** C07C 275/40

(21) Anmeldenummer : **89122771.2**

(22) Anmeldetag : **09.12.89**

## (54) Verfahren zur Herstellung von N,N'-Bis(3-aminophenyl)harnstoffen.

(30) Priorität : **23.12.88 DE 3843410**

(43) Veröffentlichungstag der Anmeldung :
**27.06.90 Patentblatt 90/26**

(45) Bekanntmachung des Hinweises auf die Patenterteilung :
**09.09.92 Patentblatt 92/37**

(84) Benannte Vertragsstaaten :
**AT BE CH DE ES FR GB IT LI NL**

(56) Entgegenhaltungen :
**US-A- 1 617 847**
**US-A- 2 503 797**

(73) Patentinhaber : **BAYER AG**
**W-5090 Leverkusen 1 Bayerwerk (DE)**

(72) Erfinder : **Ruckes, Andreas, Dr.**
**Herderstrasse 13**
**W-5090 Leverkusen 3 (DE)**
Erfinder : **Grögler, Gerhard, Dr.**
**von-Diergardt-Strasse 46**
**W-5090 Leverkusen 1 (DE)**
Erfinder : **Kopp, Richard, Dr.**
**Bilharzstrasse 15**
**W-5000 Köln 80 (DE)**

**EP 0 374 653 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein einfaches Verfahren zur Herstellung von N,N'-Bis(3-aminophenyl)harnstoffen der allgemeinen Formel (I), die gegebenenfalls geringe Mengen an oligomeren Harnstoffen der allgemeinen Struktur (II) enthalten können.

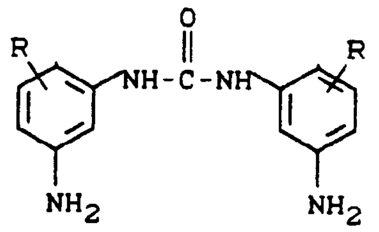

(I)

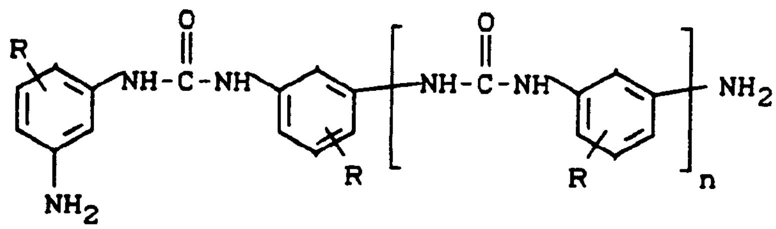

(II)

(R = lineare oder verzweigte Alkylgruppe mit 1 bis 6 C-Atomen, die in 2-, 4- und/oder 6-Stellung stehen können).

Das erfindungsgemäße Verfahren beinhaltet die Umsetzung von Ortho-alkylsubstituierten m-Phenylendiaminen mit Harnstoff in Chlorbenzol als Lösungsmittel.

Die aus dem erfindungsgemäßen Verfahren erhaltenen Diaminodiphenylharnstoffe können in fester oder gelöster Form als Kettenverlängerer zur Herstellung von Polyurethanpolyharnstoff-Elastomeren bzw. reinen Polyharnstoff-Elastomeren verwendet werden. Die erfindungsgemäßen Diaminodiphenylharnstoffe können z.B. auch als Kopplungskomponente für Diazofarbstoffe, als Härter für Epoxid- und Phenolharze sowie für alle anderen an sich bekannten Reaktionen von Aminen wie Amid- oder Imidbildung und andere verwendet werden.

Es ist bekannt, Diaminodiphenylharnstoffe durch Phosgenierung entsprechender Nitroaniline zum Dinitrodiphenylharnstoff und anschließender katalytischer Reduktion zum Diamin zu erhalten, wie z.B. die von I.L. Khmel'nitskaya et al. im Zh. Obsh. Khim 30 (2) (1960) auf der S. 602 beschriebene Synthese von III oder die vom W.R. Turner und L.M. Werbel im J. Med Chem. 28 (1985) auf der S. 1738 beschriebene Synthese des N,N'-Bis(5-amino-2-methylphenyl)-harnstoffes IV.

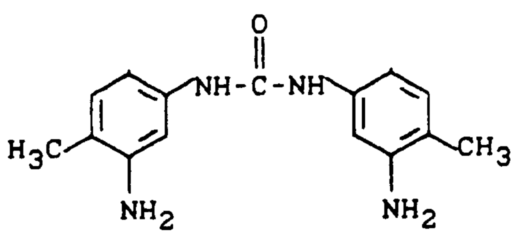

(III)

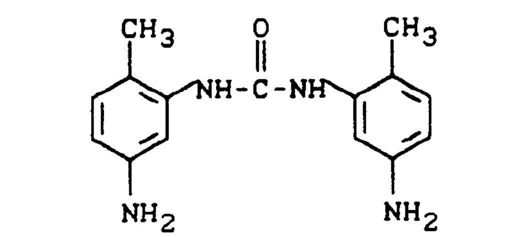

(IV)

Nachteilig ist neben den i.a. schlechten Ausbeuten der Gesamtreaktion vor allem der kostenintensive Reduktionsschritt.

Eine weitere zweistufige Synthese zur Herstellung von Diaminodiphenylharnstoffen ist die von H. Schiff und A. Ostrogovick in Liebigs Ann., 293 1896 auf den Seiten 371 ff. am Beispiel des N,N'-Bis(4-aminophenyl)harnstoffes beschriebene Umsetzung von N-Acetyl-p-phenylendiamin mit Harnstoff und anschließender Verseifung der Acetyl-Schutzgruppe. Nachteilig hierbei ist, daß monoacetylierte Diamine, die in der Regel nicht einfach herstellbar sind, als Ausgangskomponenten verwendet werden müssen.

Spezielle p-Phenylendiamine, in denen die Reaktivität einer $NH_2$-Gruppe durch geeignete o-Substituenten stark erniedrigt ist, können mit Phosgen direkt zu den entsprechenden Aminocarbaniliden umgesetzt werden. Geeignete Diamine sind z.B. 2,5-Diaminosulfonsäure (DRP-140613, Frl. 11, 1292) oder 2,6-Dichlor-p-phenylendiamin (DRP-268658, Frl. 11, 164). Andere Phenylendiamine ergeben unter solchen Bedingungen praktisch ausschließlich Polyharnstoffe, die als reaktive Kettenverlängerer völlig ungeeignet sind.

Ein einfaches Einstufenverfahren ist die in der US-PS 2 503 797 beschriebene Reaktion von p-Phenylendiamin mit Harnstoff in wäßriger Lösung. Man erhält in hoher Ausbeute praktisch reinen N,N-Bis(4-aminophenyl)harnstoff. Werden allerdings m-Phenylendiamine als Ausgangsprodukte verwendet, können die entsprechenden Harnstoffe nach der Lehre der obengenannten Patentschrift nur dann oligomerenarm und in guter Ausbeute erhalten werden, wenn 4 Äquivalente Schwefelsäure zugesetzt werden. Zur Aufarbeitung muß das ausgefallene schwefelsaure Salz des Harnstoffs mit $BaCl_2$ in das entsprechende Chlorid umgewandelt werden, aus dem dann die freie Base erhalten werden kann.

Schließlich lehrt die US-PS 1 617 847 die Herstellung von N,N'-Bis(4-aminophenyl)harnstoffen durch Reaktion von p-Phenylendiamin und alkylsubstituierten p-Phenylendiaminen mit Harnstoff in Substanz oder in inerten Lösungsmitteln, wie z.B. o-Dichlorbenzol. Wird jedoch 2,4-Diaminobenzol (TDA-2,4) unter den in den Patentbeispielen angegebenen Bedingungen eingesetzt, erhält man lediglich einen Oligoharnstoff mit niedriger NH-Zahl, der für die beabsichtigten Anwendungen völlig ungeeignet ist.

Zusammenfassend kann gesagt werden, daß alle bisher bekannten Verfahren entweder aufwendig sind, nur für spezielle Diamine geeignet sind oder nur mit p-substituierten Phenylendiaminen als Ausgangsverbindungen zu den gewünschten niedermolekularen Harnstoffen führen.

Aufgabe dieser Erfindung war es, eine einfache Synthese für Harnstoffe auf der Basis von ortho-alkylsubstituierten m-Phenylendiaminen zu finden.

Zwei Aspekte sind dabei besonders zu beachten. Auf der einen Seite sollte das Verfahren zu möglichst niedermolekularen Harnstoffen führen, da wie schon erwähnt Polyharnstoffe aufgrund zu geringer Reaktivität nicht mehr als Kettenverlängerer geeignet sind. Auf der anderen Seite sollte der Anteil an monomerem Ausgangsamin in den Harnstoffen so weit wie möglich reduziert sein, um jegliche physiologische Gefährdung beim Umgang mit dem Produkt sowie dem bekannten negativen Einfluß von freien, aromatischen, niedermolekularen Aminen auf die Licht- und Verfärbungsstabilität der damit hergestellten PUR-Kunststoffe auszuschalten.

Überraschenderweise gelang die Lösung dieser Aufgabe durch die Reaktion von ortho-alkylsubstituierten m-Phenylendiaminen mit Harnstoff in Chlorbenzol als Lösungsmittel. Die Reaktion muß in einem bestimmten Phenylendiamin/Harnstoff-Molverhältnis >2:1 durchgeführt werden.

Gegenstand der Erfindung ist somit ein Verfahren zur Herstellung von N,N'-Bis(3-aminophenyl)harnstoffen der allgemeinen Formel (I), worin

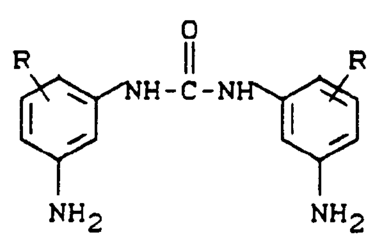

(I)

R eine lineare oder verzweigte Alkylgruppe mit 1 bis 6 Kohlenstoffatomen bedeutet, die sich in 2-, 4- und/oder 6-Stellung befinden kann, dadurch gekennzeichnet, daß die entsprechenden Phenylendiamine mit Harnstoff in einem Molverhältnis >2:1 in Chlorbenzol als Lösungsmittel umgesetzt werden.

Im folgenden werden die Verfahrensbedingungen genauer erläutert.

Besonders geeignet für das Verfahren sind 1-Alkyl-2,4-diaminobenzole, wie z.B. Toluylen-2,4-diamin. Die den Ansprüchen genügenden Verbindungen können entweder allein oder auch in Kombination miteinander eingesetzt werden.

Als Lösungsmittel ist ausschließlich Chlorbenzol geeignet. Verwendet man andere Lösungsmittel als Chlorbenzol, wie z.B. o-Dichlorbenzol oder Xylol, so erhält man entweder hochmolekulare Harnstoffe oder die Produkte enthalten bedingt durch beim Ausfallen des Harnstoffes mitgerissenes 2,4-Diaminotoluol einen bedenklich hohen Anteil an monomerem Ausgangsamin, welches nur durch relativ aufwendige Reinigungsoperationen (z.B. Umkristallisieren o.ä.) entfernt werden kann.

m-Phenylendiamin und Harnstoff werden in einem Molverhältnis >2:1, bevorzugt >3:1 bis 10:1, besonders bevorzugt >3:1 bis 5:1 eingesetzt. Werden Molverhältnisse = <2:1 gewählt, erhält man Produkte mit zu niedrigen NH-Zahlen, d.h. stark vorverlängerte Harnstoffe. Im besonders bevorzugten Bereich werden praktisch Produkte mit NH-Zahlen nahe der Theorie erhalten. Erhöhung des Molverhältnisses ist prinzipiell möglich, führt aber auf der einen Seite zu keiner Verbesserung des Produktes und ist auf der anderen Seite auch wirtschaftlich nicht sinnvoll.

Die m-Phenylendiamine werden in Mengen zwischen 20 und 200 Gew.-Teilen, vorzugsweise zwischen 40 und 150 Gew.-Teilen und besonders bevorzugt zwischen 80 und 120 Gew.-Teilen, bezogen auf 100 Gew.-Teile Lösungsmittel, im Chlorbenzol gelöst. Höhere Verdünnungen sind im Prinzip möglich, man muß jedoch zunehmende Vorverlängerung in Kauf nehmen. Wird auf der anderen Seite die Konzentration zu stark erhöht, so ist der Reaktionsansatz durch ausfallendes Produkt zunehmend schlechter rührbar und zusätzlich enthält das Produkt erhebliche Mengen an mitgerissenem Ausgangsamin, welches nur durch aufwendige Reinigungsoperationen entfernt werden kann. Mit anderen Worten kann man durch Wählen der Konzentration des Ausgangsamin in gewissen Grenzen die NH-Zahl des gewünschten Harnstoffes steuern. Im bevorzugten Konzentrationsbereich werden NH-Zahlen nahe der Theorie erhalten. Zu der Lösung des Amins im Chlorbenzol 1 Mol wird der Harnstoff im beschriebenen Molverhältnis gegeben und die Reaktionsmischung bei Temperaturen zwischen 60 und 136°C, bevorzugt unter Rückfluß, gerührt.

Das ausgefallene Produkt wird abfiltriert und zum Entfernen restlicher Mengen Ausgangsamin mit Chlorbenzol gewaschen. Von Vorteil erweist sich hierbei, vor dem Filtrieren Chlorbenzol zuzufügen und die Filtration in einer mit Dampf beheizten Filternutsche durchzuführen, um ein Ausfallen des Restamin bei niedrigen Temperaturen zu verhindern. Ebenso ist es aus den gleichen Gründen vorteilhaft mit heißem Chlorbenzol nachzuwaschen. Das aus der Mutterlauge gewinnbare überschüssige Ausgangsamin kann ohne weitere Reinigung wiederverwendet werden.

Nach dem Trocknen erhält man den gewünschten Harnstoff als feinkristallinen Feststoff in ausgezeichneter Ausbeute und mit NH-Zahlen, abhängig von den gewählten Konzentrationsverhältnissen, von >270 mg KOH/g (th. 416 mg KOH/g), vorzugsweise von >360 mg KOH/g und besonders bevorzugt >380 mg KOH/g, wobei der Gehalt an freiem monomerem Ausgangsamin <1 Gew.-%, vorzugsweise <0,5 Gew.-% beträgt.

Die nachfolgenden Beispiele erläutern das erfindungsgemäße Verfahren, ohne es jedoch einzuschränken (%-Angaben bedeuten Gew.%, soweit nicht anderes vermerkt wurde).

## Beispiel 1

40 kg (327,8 Mol) Toluylen-2,4-diamin (TDA-2,4; Fp.: 98°C) und 4920 g (82 Mol) Harnstoff (TDA-2,4/Harnstoff 4:1) werden bei Raumtemperatur zu 44 kg Chlorbenzol (90 Gew.-Teile TDA-2,4, bezogen auf 100 Gew.-Teile Chlorbenzol) gegeben. Unter Inertgasatmosphäre ($N_2$) wird die Reaktionsmischung für 19 Stunden unter Rückfluß gerührt. Nach dem Verdünnen mit 140 kg Chlorbenzol wird die Reaktionsmischung weitere 60 Minuten unter Rückfluß erhitzt. Das ausgefallene Produkt wird bei ca. 100°C durch eine heizbare Nutsche abfiltriert, 4 mal mit je 50 kg heißem Chlorbenzol gewaschen und anschließend bei 80°C im Vakuumtrockenschrank getrocknet.

Ausbeute: 19,5 kg (89 %, bezogen auf eingesetzten Harnstoff).
NH-Zahl ($HClO_4$/Eisessig): 406 mg KOH/g (th. 416 mg KOH/g).
TDA-2,4-Restgehalt (HPLC): 0,253 Gew.-%.

## Beispiel 2

366 g (3 Mol) Toluylen-2,4-diamin (TDA-2,4) und 51,4 g (0,86 Mol) Harnstoff (TDA-2,4/Harnstoff 3,48:1) werden bei Raumtemperatur zu 366 g Chlorbenzol (100 Gew.-Teile TDA-2,4, bezogen auf 100 Gew.-Teile Chlorbenzol) gegeben. Die Reaktionsmischung wird 19 Stunden unter Rückfluß gerührt. Nach dem Verdünnen mit 1208 g Chlorbenzol wird die Reaktionsmischung weitere 30 Minuten unter Rückfluß erhitzt. Das ausgefallene Produkt wird bei ca. 100°C durch eine mit Dampf beheizte Nutsche abfiltriert, mit ca. 500 ml heißem Chlorbenzol gewaschen und anschließend bei 80°C im Vakuumtrockenschrank getrocknet.

Ausbeute 199 g (86 %, bezogen auf eingesetzten Harnstoff).
NH-Zahl ($HClO_4$/Eisessig): 405 mg KOH/g (th. 416 mg KOH/g).

TDA-2,4-Restgehalt (HPLC): 0,285 Gew.-%.

Beispiel 3

260 g (2,13 Mol) Toluylen-2,4-diamin (TDA-2,4) und 49 g (0,814 Mol) Harnstoff (TDA-2,4/Harnstoff 2,62:1) werden bei Raumtemperatur zu 1334 g Chlorbenzol (19,5 Gew.-Teile TDA-2,4, bezogen auf 100 Gew.-Teile Chlorbenzol) gegeben. Die Reaktionsmischung wird für 12 Stunden unter Rückfluß gerührt. Das ausgefallene Produkt wird heiß abfiltriert, mit Toluol und Petrolether gewaschen und anschließend im Vakuumtrockenschrank getrocknet.

Ausbeute: 169 g (76,5 %, bezogen auf eingesetzten Harnstoff).
NH-Zahl ($HClO_4$/Eisessig): 336 mg KOH/g
TDA-2,4-Restgehalt (HPLC): 0,1 Gew.-%.

Beispiel 4

350 g (2,86 Mol) Toluylen-2,4-diamin (TDA-2,4) und 43 g (0,716 Mol) Harnstoff (TDA-2,4/Harnstoff 4:1) werden bei Raumtemperatur zu 778 g Chlorbenzol (45 Gew.-Teile TDA-2,4, bezogen auf 100 Gew.-Teile Chlorbenzol) gegeben. Die Reaktionsmischung wird für 19 Stunden unter Rückfluß gerührt. Nach Zugabe von ca. 1600 g Chlorbenzol wird das ausgefallene Produkt heiß abfiltriert und anschließend im Vakuumtrockenschrank getrocknet.

Ausbeute: 170 g (85 %, bezogen auf eingesetzten Harnstoff).
NH-Zahl ($HClO_4$/Eisessig): 407 mg KOH/g.
TDA-2,4-Restgehalt (HPLC): 0,254 Gew.-%.

Beispiel 5a

1800 g (14,75 Mol) Toluylen-2,4-diamin (TDA-2,4) und 221 g (3,68 Mol) Harnstoff (TDA-2,4/Harnstoff 4:1) werden bei Raumtemperatur zu 2000 g Chlorbenzol (90 Gew.-Teile TDA-2,4, bezogen auf 100 Gew.-Teile Chlorbenzol) gegeben. Die Reaktionsmischung wird für 19 Stunden unter Rückfluß gerührt. Nach Zugabe von 6670 g Chlorbenzol wird eine weitere Stunde unter Rückfluß gerührt. Das ausgefallene produkt wird heiß durch eine heizbare Nutsche abfiltriert, mit ca. 2 l heißem Chlorbenzol gewaschen und anschließend im Vakuumtrockenschrank getrocknet.

Ausbeute: 910 g (91,5 %, bezogen auf eingesetzten Harnstoff).
NH-Zahl ($HClO_4$/Eisessig): 414 mg KOH/g.
TDA-2,4-Restgehalt (HPLC): 0,417 Gew.-%.

Die Mutterlauge wurde bis auf ca. 1900 g eingeengt. Das wiedergewonnene Chlorbenzol wurde in 5b) als Lösungsmittel verwendet.

Beispiel 5b (der Ansatz 5a) wurde wiederholt):

Ausbeute: 856 g (91,5 %, bezogen auf eingesetzten Harnstoff).
NH-Zahl ($HClO_4$/Eisessig): 395 mg KOH/g.
TDA-2,4-Restgehalt (HPLC): 0,291 Gew.-%.

Die Mutterlauge wurde bis auf ca. 1900 g eingeengt und mit der eingeengten Mutterlauge aus 5a) vereinigt. Der Gehalt an Toluylen-2,4-diamin beträgt ca. 1800 g.

Zu den vereinigten Mutterlaugen aus a) und b) wurden bei Raumtemperatur 221 g Harnstoff zugesetzt und die Reaktionsmischung 19 Stunden unter Rückfluß gerührt. Nach Zugabe von ca. 6500 g aus a) und b) wiedergewonnenem Chlorbenzol wird für eine weitere Stunde unter Rückfluß gerührt. Die weitere Aufarbeitung erfolgt wie unter a) beschrieben.

Ausbeute: 1012 g (102 %, bezogen auf eingesetzten Harnstoff).

Die Ausbeute >100 % erklärt sich dadurch, daß in den Mutterlaugen aus a) und b) noch gelöstes Produkt enthalten ist, welches in der Folgereaktion ausfällt.

NH-Zahl ($HClO_4$/Eisessig): 390 mg KOH/g.
TDA-2,4-Restgehalt (HPLC): 0,158 Gew.-%.

Beispiel 6 (Vergleich)

(Weniger bevorzugte Reaktionsbedingungen)

200 g (1,64 Mol) Toluylen-2,4-diamin (TDA-2,4) und 49 g (0,816 Mol) Harnstoff (TDA-2,4/Harnstoff 2,01:1) werden bei Raumtemperatur zu 1334 g Chlorbenzol (15 Gew.-Teile TDA-2,4, bezogen auf 100 Gew.-Teile Chlorbenzol) gegeben. Die Reaktionsmischung wird für 5 Stunden unter Rückfluß gerührt. Das ausgefallene Produkt wird heiß abfiltriert und mit Petrolether gewaschen.

Ausbeute 90 g (41 %, bezogen auf eingesetzten Harnstoff).

NH-Zahl ($HClO_4$/Eisessig): 274 mg KOH/g (th. 416 mg KOH/g).

## Patentansprüche

1. Verfahren zur Herstellung von N,N'-Bis(3-aminophenyl)harnstoffen der allgemeinen Formel (I), worin

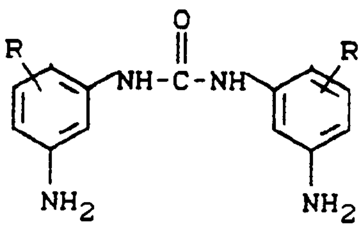

(I)

R eine lineare oder verzweigte Alkylgruppe mit 1 bis 6 Kohlenstoffatomen bedeutet, die sich in 2-, 4- und/oder 6-Stellung befinden kann, dadurch gekennzeichnet, daß die entsprechenden Phenylendiamine mit Harnstoff in einem Molverhältnis >2:1 in Chlorbenzol als Lösungsmittel umgesetzt werden.

2. Verfahren nach Anspruch 1, dadurch gekennzeichnet, daß das Molverhältnis von m-Phenylendiamin zu Harnstoff >3:1 beträgt.

3. Verfahren nach den Ansprüchen 1 und 2, dadurch gekennzeichnet, daß sich der Alkylsubstituent in 4- und/oder 6-Stellung befindet.

4. Verfahren nach den Ansprüchen 1 und 2, dadurch gekennzeichnet, daß der Alkylsubstituent eine Methyl-Gruppe ist.

## Claims

1. A process for the production of N,N'-bis-(3-aminophenyl)-ureas corresponding to general formula (I)

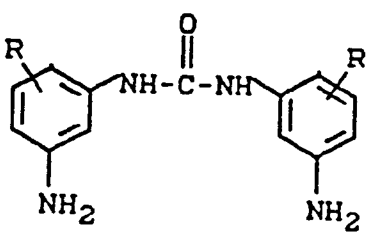

(I)

in which
R is a linear or branched $C_1$-$C_6$ alkyl group which may be in the 2-, 4- and/or 6-position,
characterized in that the corresponding phenylene diamines are reacted with urea in a molar ratio of > 2:1 in chlorobenzene as solvent.

2. A process as claimed in claim 1, characterized in that the molar ratio of m-phenylene diamine to urea is > 3:1.

3. A process as claimed in claims 1 and 2, characterized in that the alkyl substituent is in the 4- and/or 6-position.

4. A process as claimed in claims 1 and 2, characterized in that the alkyl substituent is a methyl group.

## Revendications

1. Procédé de production de N,N'-bis(3-aminophényl)urées de formule générale (I)

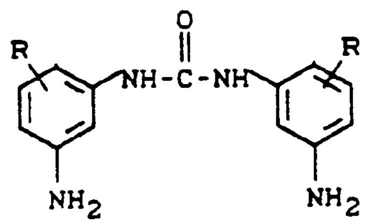

(I)

dans laquelle
R est un groupe alkyle linéaire ou ramifié ayant 1 à 6 atomes de carbone, qui peut se trouver en position 2, 4 et/ou 6, caractérisé en ce que les phénylène-diamines correspondantes sont amenées à réagir avec l'urée dans un rapport molaire supérieur à 2:1 dans du chlorobenzène utilisé comme solvant.

2. Procédé suivant la revendication 1, caractérisé en ce que le rapport molaire de la m-phénylènediamine à l'urée est supérieur à 3:1.

3. Procédé suivant les revendications 1 et 2, caractérisé en ce que le substituant alkyle se trouve en position 4 et/ou 6.

4. Procédé suivant les revendications 1 et 2, caractérisé en ce que le substituant alkyle est un groupe méthyle.